(19) Europäisches Patentamt
European Patent Office
Office européen des brevets

(11) **EP 3 524 980 A1**

(12) **EUROPEAN PATENT APPLICATION**

(43) Date of publication:
14.08.2019 Bulletin 2019/33

(51) Int Cl.:
***G01N 33/50*** *(2006.01)*

(21) Application number: **18208719.7**

(22) Date of filing: **20.05.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **20.05.2009 PCT/US2009/044751**

(62) Document number(s) of the earlier application(s) in accordance with Art. 76 EPC:
**10721224.3 / 2 433 125**

(71) Applicants:
• **Mayo Foundation for Medical Education and Research**
**Rochester, MN 55905 (US)**
• **Cardio3 BioSciences S.A.**
**1435 Mont-Saint-Guibert (BE)**

(72) Inventors:
• **GORDON-BERESFORD, Roland**
**B-1310 La Hulpe (BE)**

• **GAUSSIN, Vinciane**
**B-1970 Wezembeek-Oppem (BE)**
• **HOMSY, Christian**
**B-1150 Bruxelles (BE)**
• **TERZIC, Andre**
**Rochester, MN 55902 (US)**
• **BEHFAR, Atta**
**Rochester, MN 55902 (US)**

(74) Representative: **Crease, Devanand John et al**
**Keltie LLP**
**No.1 London Bridge**
**London SE1 9BA (GB)**

Remarks:
•This application was filed on 27-11-2018 as a divisional application to the application mentioned under INID code 62.
•Claims filed after the date of filing of the application / after the date of receipt of the divisional application (Rule 68(4) EPC).

(54) **METHOD FOR DETERMINING THE CARDIO-GENERATIVE POTENTIAL OF MAMMALIAN CELLS**

(57) This document is related to a method for determining the cardio-generative potential of mammalian cells which comprises the assessment of a CARdiac generation Potential Index (CARPI) as a function of the quantification of the expression of genes of said cells. It also relates to a method for quantitatively assessing the modification of this cardio-generative potential and the cardiogenic potential of a treatment aiming at cellular differentiation.

Fig. 1

**Description**

**CROSS-REFERENCE TO RELATED APPLICATIONS**

This application claims the benefit of priority to International Application Serial No. PCT/US2009/044751, filed May 20, 2009. The disclosure of the prior application is considered part of (and is incorporated by reference in) the disclosure of this application.

**Field of the invention**

The present invention relates to the treatment of heart disease disorders through injection of mammalian cells. In particular, it relates to a method for quantitatively assessing the cardio-generative potential of mammalian cells, thereby allowing a good predictability of the success of repairing a heart in need. It also relates to a method for quantitatively assessing the modification of this cardio-generative potential and the cardiogenic potential of a treatment aiming at cellular differentiation, and a computer device comprising a processor, and a memory encoding one or more non-neural network programs coupled to the processor, wherein said programs cause the processor to perform a method, said method comprising calculating a CARPI.

**State of the art**

Cardiovascular diseases are leading cause of morbidity and mortality worldwide, despite advances in patient management. In contrast to tissues with high reparative capacity, heart tissue is vulnerable to irreparable damages. Cell-based regenerative cardiovascular medicine is now being pursued in the clinical setting to address heart disease disorders.

Recent advent of stem cell biology extends the scope of current models of practice from traditional palliation towards curative repair. Typically, clinical experience has been based on adult stem cells delivered in an unaltered state. First generation biologics are naive human stem cells, identified as readily accessible cytotypes. It has been shown that a few individuals improve on delivery of naive human stem cells. The state of the art in the field of naive cell transplantation in the heart of humans was described inter alia in the review carried by Abdel-Latif A. et al. 'Adult bone marrow-derived cells for cardiac repair: a systematic review and meta-analysis.' Arch Intern Med. (2007) 167:989-997, and citations therein.

To improve clinical outcome, second-generation stem cell therapies were developed to guide naive human stem cells towards the cardiac lineage prior to injection into the patient. In the review by Behfar et al. 'Guided stem cell cardiopoietic: Discovery and translation' J. Mol. and Cell. Cardiology (2008) 45: 523-529, the concept of using cardiac precursor cells, such as cardiopoietic cells, for heart regeneration was discussed.

Cardiopoietic cells have a unique phenotype: they are characterized by nuclear translocation of Nkx2.5 and MEF2C polypeptides, combined to the absence of detectable sarcomeric proteins. This cardiopoietic status corresponds to an intermediate cell phenotype, i.e. committed to the cardiac lineage but not yet fully differentiated. Non-detectable level of sarcomeric protein expression is a unique feature of cardiopoietic cells which distinguishes them from contractile and sarcomeric-containing cardiomyocyte-like cells derived from stem cells and described in other applications such as by Chunhui Xu (US 2005/0164382) and Lough et al (US 2002/0061837).

Increased protein content of a transcription factor may not imply its subcellular localization, which could be either cytoplasmic or nuclear. Nuclear translocation of Nkx2.5 and MEF2C polypeptides is necessary for definitive cardiac lineage commitment. This is further explained in Behfar A. et al, (Derivation of a cardiopoietic population from human mesenchymal stem cells yields cardiac progeny, Nature Clinical Practice, 2006, 3:S78-S82). Although nuclear translocation may be qualitatively observed by immunocytochemistry or immunohistochemistry, techniques such as western blotting or Fluorescence Activated Cell Sorting (FACS) that look at total protein content are not suitable for quantitative assessment of the subcellular distribution of a polypeptide. The observation of subcellular distribution of a polypeptide, as described in US 2008/0019944, is not only qualitative but also time-consuming in the industrial perspective and operator-dependent. Thus clinical outcome, i.e. the cardio-generative potential of these "first-generation" naive stem cells and "second-generation" guided stem cells could not be readily predicted prior to injection.

A method to quantitatively assess the cardio-generative potential of mammalian cells remained to be proposed.

The present invention now provides such a predictive method for determining the cardio generative potential of mammalian cells which comprises the quantitative assessment of a CARdiac generation Potential Index (CARPI) as a function of the quantification of the expression of genes of said cells. It also addresses the quantitative assessment of the modification of the cardio generative potential of mammalian cells and the cardiogenic potential of a treatment aiming at cellular differentiation.

**Definitions**

Within the frame of the present document, and unless indicated to the contrary, the terms designated below between quotes have the following definitions.

The *'cardio-generative potential'* of a cell designates the ability of this cell to succeed to generate heart cells, for instance cardiac myocytes.

*'Cardiopoietic cells'* are an intermediate cell phenotype, i.e. committed to the cardiac lineage but not yet fully differentiated. Cardiopoietic cells are characterized by nuclear translocation of Nkx2.5 and MEF2C, combined to the absence of detectable sarcomeric proteins (Behfar et al. 'Derivation of a cardiopoietic population from human mesenchymal stem yields progeny', Nature Clin. Pract., Cardiovasc. Med. (2006) 3: S78-S82). Cardiopoietic cells retain a proliferative capacity. Cardiopoietic cells can be derived from stems cells including for example, human adult mesenchymal stem cells (Terzic et al. US 2008/0019944), mouse embryonic stem cells (Behfar et al, 'Cardiopoietic programming of embryonic stem cells for tumour-free heart repair' J Exp Med 2007 204: 405-420), embryonic-like stem cells, inducible pluripotent stem cells, umbilical cord blood cells, resident cardiac stem cells and the like, or any other adapted source (provided their production implies no human embryo destruction).

A *'cocktail'* or *'cardiogenic cocktail'* designates a composition containing at least two cardiogenic substances.

A *'cardiogenic treatment'* is a treatment which improves the cardio-generative potential of a cell. Example of such treatment consists in putting said cell in contact with a cocktail. Examples of such cocktails comprise at least two substances selected in the group consisting of growth factors, cytokines, hormones and combinations thereof. Said at least two substances may be selected in the group consisting of bone morphogenetic proteins (BMP) such as BMP-1, BMP-2, BMP-5, BMP-6; epidermal growth factor (EGF); erythropoietin (EPO); fibroblast growth factors (FGF) such as FGF-1, FGF-4, FGF-5, FGF-12, FGF-13, FGF-15, FGF-20; granulocyte-colony stimulating factor (G-CSF); granulocyte-macrophage colony stimulating factor (GM-CSF); growth differentiation factor-9 (GDF-9); hepatocyte growth factor (HGF); insuline-like growth factor (IGF) such as IGF-2; myostatin (GDF-8); neurotrophins such as NT-3, NT-4, NT-1 and nerve growth factor (NGF); platelet-derived growth factor (PDGF) such as PDGF-beta, PDGF-AA, PDGF-BB; thrombopoietin (TPO); transforming growth factor alpha (TGF-$\alpha$); transforming growth factors $\beta$ (TGF-$\beta$) such as TGF-$\beta$1, TGF-$\beta$2, TGF-$\beta$3; vascular endothelial growth factor (VEGF) such as VEGF-A, VEGF-C; TNF-$\alpha$; leukemia inhibitory factor (LIF); interleukin 6 (IL-6); retinoic acid; stromal cell-derived factor-1 (C SDF-1); brain-derived neurotrophic factor (BDNF); periostin; angiotensin II; Flt3 ligand; glial-derived neurotrophic factor; heparin; insulin-like growth factor binding protein-3; insulin-like growth factor binding protein-5; interleukin-3; interleukin-8; midkine; progesterone; putrescine; stem cell factor; Wnt1; Wnt3a; Wnt5a; caspase-4; chemokine ligand 1; chemokine ligand 2; chemokine ligand 5; chemokine ligand 7; chemokine ligand 11; chemokine ligand 20; haptoglobin; lectin; cholesterol 25-hydroxylase; syntaxin-8; syntaxin-11; ceruloplasmin; complement component 1; complement component 3; integrin alpha 6; lysosomal acid lipase 1; $\beta$-2 microglobulin; ubiquitin; macrophage migration inhibitory factor; cofilin; cyclophillin A; FKBP12; NDPK; profilin 1; cystatin C; calcyclin; stanniocalcin-1; PGE-2; mpCCL2; IDO; iNOS; HLA-G5; M-CSF; angiopoietin; PlGF; MCP-1; extracellular matrix molecules; CCL2 (MCP-1); CCL3 (MIP-1$\alpha$); CCL4 (MIP-1$\beta$); CCL5 (RANTES); CCL7 (MCP-3); CCL20 (MIP-3$\alpha$); CCL26 (eotaxin-3); CX3CL1 (fractalkine); CXCL5 (ENA-78); CXCL11 (i-TAC); CXCL1 (GRO$\alpha$); CXCL2 (GRO$\beta$); CXCL8 (IL-8); CCL10 (IP-10); and combinations thereof.

A *'cocktail-guided cell'* or a '*cell guided towards cardiac differentiation'* is a cell which has been put into contact with a cocktail.

*'Differentiation'* is the process by which a less specialized cell becomes a more specialized cell.

*'Ejection fraction'* means the fraction of blood pumped out during a heartbeat. Without a qualifier, the term ejection fraction refers specifically to that of the left ventricle (left ventricular ejection fraction or LVEF).

As used in the subject specification, the singular forms 'a', 'an' and *'the'* include plural aspects unless the context clearly dictates otherwise. Thus, for example, reference to *'a stem cell'* includes a single cell, as well as two or more cells; reference to *'an agent'* or 'a *reagent'* includes a single agent or reagent, as well as two or more agents or reagents; reference to 'the invention' or 'an invention' includes single or multiple aspects of an invention; and so forth.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

**Summary of the invention**

The invention provides a method for determining the cardio-generative potential of mammalian cells or cardiogenic potential of a treatment which comprises the assessment of a CARdiac generation Potential Index (CARPI) as a function of the quantification of the expression of genes of said cells.

Preferably, the CARPI is a function of the quantification of messenger RNA (mRNA) levels of specific genes of said cells.

Preferably, at least one gene is chosen from the group consisting of Nkx2.5, Tbx5, MEF2C, GATA4, GATA6, Mesp1, FOG1, FOG2, Flk1, homologues thereof in mammals and combinations of these genes. The cells may be cardiac progenitor cells. They may also be somatic, germ, umbilical cord blood, cardiac progenitor, embryonic, and/or genetically modified cells.

In some cases, the cells can belong to one individual, and a CARPI can be assessed for those cells before and after exposing the cells to any cardiogenic treatment.

In another embodiment, a CARPI is assessed for cells of an individual or group of individuals *versus* another individual or group of individuals.

In a method particularly preferred, the CARPI is a multivariate equation where the expression of genes at the mRNA level is quantified as variables.

The equation is preferably chosen from the group consisting of polynomials functions, transcendental functions, and combinations thereof.

In a particular embodiment of a method provided herein a CARPI is measured to quantitatively assess the cardiogenic potential of a treatment.

According to one embodiment of a method provided herein, the CARPI may be put into correlation with a parameter of cardiac function.

The invention also relates to a computer device comprising a processor, and a memory encoding one or more programs coupled to the processor, wherein the one or more programs cause the processor to perform a method, said method comprising calculating a CARPI.

**Brief Description of the Drawing**

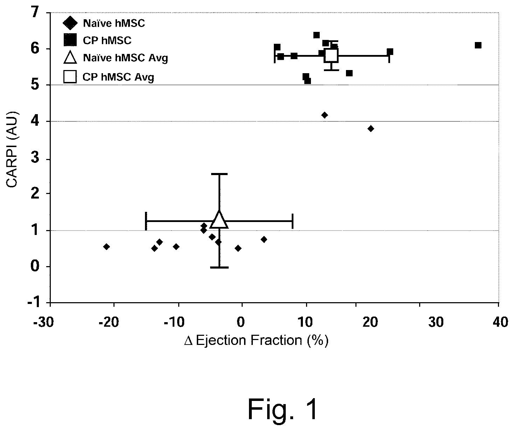

Fig. 1 shows in Y ordinate the CARPI, in arbitrary units (AU), calculated for both naive human MSC (hMSC) and cocktail guided-hMSC (CP-hMSC) on the basis of quantification of the expression of genes at the mRNA level and in X ordinate the change of LVEF ($\Delta$EF) in percent prior and after injection in mouse infarcted hearts. Black symbols represent individual data; open symbols represent averaged data (Avg).

**Detailed description of the invention**

**Example 1**

Bone marrow samples were harvested from patients undergoing coronary artery bypass for ischemic heart disease. Patients provided informed consent, as approved by competent Institutional Ethics Committees.

Mesenchymal stem cells were recruited by plating of raw bone marrow on plastic dishes, with a wash at 12h, selecting adhesive cells with identity confirmed by Fluorescence-Activated Cell Sorting (FACS) analysis using the CD34$^-$/CD45$^-$/CD133$^+$ marker panel. Cells were further cultured and expanded at 37° C in DMEM supplemented with 5% human platelet lysate (Mayo Clinic Blood Bank, Rochester, MN).

Naive human bone marrow-derived mesenchymal stem cells were cultured in either platelet lysate or serum supplemented with a cardiogenic cocktail consisting in TGF$\beta$-1 (2.5 ng/ml), BMP4 (5 ng/ml), FGF2 (5 ng/ml), IGF-1 (50 ng/ml), Activin-A (10 ng/ml), Cardiotrophin (1 ng/ml), $\alpha$-thrombin (1 U/ml), and Cardiogenol C (100 nM) in order to derive a cardiopoietic population.

The present invention allows the quantitative assessment of the cardio-generative potential of said cardiopoietic population, by quantifying the expression of two or more genes at the RNA level. This invention obviates the problems of qualitative observations, issue of time, and operator-dependence, inherent to the observation of subcellular location of transcription factor polypeptides. One method of choice is real-time quantitative reverse transcription polymerase chain reaction (qPCR). This method gives faster results (within one day) that are operator-independent and quantified relative to a reference standard. In addition, while immunostained samples require one-by-one fluorescent microscopy evaluation, up to 48 different samples (or conditions) can be tested in duplicate by qPCR using 96-well plates.

In order to identify suitable markers for qPCR, mRNA was extracted from cardiopoietic cells that were evaluated by immunofluorescence staining.

The reference standard consisted of cells from the same batch cultured in the absence of the cardiogenic cocktail.

Genes listed in Table 1, which are representative of cardiac transcriptional activity were evaluated.

qPCR was performed using a TaqMan PCR kit with an Applied Biosystems 7,900HT Sequence Detection System (Applied Biosystems, Foster City, CA). TaqMan Gene Expression reactions were incubated in a 96-well plate and run in triplicate. The threshold cycle ($C_T$) was defined as the fractional cycle number at which fluorescence passes a fixed threshold. TaqMan $C_T$ values were converted into relative fold changes determined using the $2^{-\Delta\Delta C_T}$ method, normalized to a housekeeping gene expression, i.e. *GAPDH* (P/N 435,2662-0506003).

Results for treated cells were normalized to results obtained for the corresponding reference standard.

A CARPI, which is a function of the quantification of the expression of two or more genes of said cells, was calculated as a linear average of the expression at the RNA level of Nkx2.5, Tbx-5, MEF2C, GATA-4, GATA-6, MESP-1 and FOG-1 using a calculation spreadsheet (Microsoft Excel 2007®, Microsoft Corporation). The following formula was used:

$$CARPI = \frac{1}{n} \sum_{i=1}^{i=n} RNA\,level_i$$

where '$i$' represents the selected gene and 'n' represents the total number of genes selected, with a minimum of 2. In this particular example, n=7.

The cardio-generative potential of hMSC-derived cardiopoietic cells was evaluated in nude, immunocompromised mice (Harlan, Indianapolis, IN). The protocol was approved by the competent Institutional Animal Care and Use Committee.

Myocardial infarction was performed. Following a blinded design, one month post-infarction a total of 600,000 total viable naive hMSC or 600,000 total viable hMSC-derived cardiopoietic cells, suspended in 12.5 $\mu$l of platelet lysate-free propagation medium, were injected under microscopic visualization in five epicardial sites on the anterior wall of the left ventricle (2.5 $\mu$l per injection site).

Left ventricular function and structure were serially followed by transthoracic echocardiography (Sequoia 512; Siemens, Malvern, PA and VisualSonics Inc, Toronto, Canada). Left ventricular ejection fraction (LVEF, %) was calculated as [(LVVd - LWs)/LVVd] x 100, where LWd is left ventricular end-diastolic volume ($\mu$l), and LVVs is left ventricular end-systolic volume ($\mu$l).

A change of LVEF ($\Delta$EF) was calculated as the difference between LVEF measured one month after cell injection and LVEF measured prior to cell injection.

Fig. 1 is a graph plotting the CARPI for each individual cell culture against the corresponding $\Delta$EF for the mouse injected with the respective said individual cell culture. Naive hMSC (small black diamonds) typically demonstrated a low CARPI associated with no significant improvement in myocardial function (negative $\Delta$EF) one month post-cell injection. It is worth noting rare batches of naive hMSCs innately possessing high CARPI value together with an innate regenerative potential. The average for all batches of naive hMSCs is shown by a large white triangle. hMSC-derived cardiopoietic cells (small black squares) typically demonstrated an elevated CARPI associated with robust increase in myocardial function (positive $\Delta$EF). The average for all batches of hMSC-derived cardiopoietic cells is shown by a large white square. Averages are represented together with the corresponding 95% confidence interval.

Thus, the inventors demonstrate that there is a positive correlation between an elevated CARPI of the cells to be injected and the change in ejection fraction after injection in the infarcted heart. Thus, the CARPI is a predictive index of cardio-generative potential.

Table 1

| Applied Biosystems Assay ID | Gene name | Gene symbol |
|---|---|---|
| Hs00231763_m1 | -Homeobox transcription factor or -NK2 transcription factor related, locus 5 | Nkx2.5 or NKX2-5 or NKX2.5 |
| Hs00171403_m1 | -Zinc finger cardiac transcription factor or -GATA binding protein 4 | GATA-4 or, GATA4 (AB) |
| Hs00231149_m1 | -Myocyte enhancer factor 2C | MEF2c or MEF2C |
| Hs00361155_m1 | -T-box transcription factor or -T-box 5 | Tbx5 or TBX5 |
| Hs00542350_m1 | -GATA co-factor ("Friend of GATA") or -zinc finger protein, multitype 1 | FOG 1 of FOG-1 or FOG1 |
| Hs00251489_m1 | -Helix-loop-helix transcription factor -Mesoderm posterior 1 homolog (mouse) (AB) | Mesp1 or MESP1 |
| Hs00232018_m1 | -GATA binding protein 6 (AB) | GATA-6 or GATA6 |

(continued)

| Applied Biosystems Assay ID | Gene name | Gene symbol |
|---|---|---|
| Hs00911699_m1 | -Kinase insert domain receptor (a type III receptor tyrosine kinase) | Flk-1, or FLK1 or KDR |

**Example 2**

Similar results have been observed by treating stem cells with a cocktail containing recombinant TGFβ-1 (2.5 ng/ml), BMP4 (5 ng/ml), Activin-A (5 ng/ml), FGF-2 (10 ng/ml), α-thrombin (1 U/ml), IGF-1 (50 ng/ml), Cardiotrophin (1 ng/ml) and Cardiogenol C (100 nM) used in a combinatorial fashion.

**Example 3**

Similar results have been observed by treating stem cells with a cocktail containing recombinant TGF-β1 (2.5 ng/ml), BMP-4 (5 ng/ml), Activin-A (5 ng/ml), FGF-2 (10 ng/ml), α-thrombin (1 U/ml), IGF-1 (50 ng/ml), IL-6 (100 ng/ml) and retinoic acid (1 μM) used in a combinatorial fashion.

**Other embodiments**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.
Alternative expressions of the inventive concept are set out in the following clauses:

1. A method for determining the cardio-generative potential of mammalian cells which comprises the assessment of a CARdiac generation Potential Index (CARPI) which is a function of the quantification of the expression of two of more genes of said cells.
2. The method according to clause 1, wherein said expression of genes is quantified at the level of messenger RNAs (mRNAs), functional RNAs such as microRNAs, or a combination thereof.
3. The method according to clause 2 wherein the level of mRNA expression is quantitatively measured from at least one gene selected from the group consisting of Nkx2.5, Tbx5, MEF2C, GATA4, GATA6, Mesp1, FOG1, FOG2, Flk1 homologues thereof in mammals, and any combinations thereof.
4. The method according to any of the preceding clauses, wherein said cells are selected in the group consisting of somatic, germinal, umbilical cord blood, cardiac progenitors, embryonic cells and any combination thereof.
5. The method according to any of the preceding clauses, wherein said cells are genetically modified.
6. The method according to any of the preceding clauses, wherein said cells contain no detectable sarcomeric proteins.
7. The method according to any of the preceding clauses, wherein the CARPI is assessed for said cells before and after any cardiogenic treatment.
8. The method according to clause 7, wherein the cardiogenic treatment comprises exposing said cells to a cocktail containing cardiogenic substances.
9. The method according to any of the preceding clauses, wherein said cells belong to one mammal.
10. The method according to any of clauses 1 to 8, wherein cells are from a mammal or group of mammals and the CARPI is assessed and compared to the CARPI of cells belonging to another mammal or group of mammals.
11. The method according to any of the preceding clauses, wherein the CARPI is a multivariate equation where the expression of genes at the mRNA level is quantified as variables.
12. The method according to clause 11, wherein said equation is chosen from the group consisting of polynomials functions, transcendental functions, and combinations thereof.
13. The method according to any of the preceding clauses, wherein the CARPI is measured to quantitatively assess the cardiogenic potential of a treatment.
14. The method according to any of the preceding clauses, wherein the CARPI is put into correlation with a parameter of cardiac function.
15. A computer device comprising a processor, and a memory encoding one or more non-neural network programs coupled to the processor, wherein said programs cause the processor to perform a method, said method comprising calculating a CARPI.

**Claims**

1. A method for determining the cardio-generative potential of mammalian cells which comprises the assessment of a CARdiac generation Potential Index (CARPI) which is a function of the quantification of the expression of genes of said cells, wherein at least one gene is selected from the group consisting of Nkx2.5, Tbx5, MEF2C, GATA4, GATA6, Mesp1, and FOG1, and wherein the CARPI is a multivariate equation where the expression of genes is quantified as variables.

2. A method according to any of the preceding claims, wherein the cells are cardiopoeitic.

3. A method according to any of the preceding claims, wherein the cells are genetically modified.

4. A method according to any of the preceding claims, wherein said cells belong to one individual, and a CARPI is assessed for said cells before and after exposing said cells to a cocktail containing cardiogenic substances.

5. A method according to any of claims 1 to 4, wherein CARPI is assessed for cells of an individual or group of individuals *versus* another individual or group of individuals.

6. A method according to any of claims 1 to 5, wherein the equation is chosen from the group consisting of polynomials functions, transcendental functions, and combinations thereof.

7. A method according to any of the preceding claims, wherein the expression of genes is considered at levels selected from the group consisting of mRNA levels and protein levels.

8. A method according to any of the preceding claims, wherein

   - a first CARPI is measured on cells while untreated,
   - a second CARPI is measured on cells treated with at least one substance,
   - the first CARPI and the second CARPI are compared in order to determine whether the substance is cardiogenic, the substance being considered cardiogenic when the second CARPI is superior to the first CARPI.

9. A method according to any of the preceding claims, wherein the CARPI is put into correlation with the left ventricular ejection fraction (LVEF) observed after the cells have been injected into the infarcted heart of a model.

10. A computer device comprising a processor, and a memory encoding one or more programs coupled to the processor, wherein the one or more programs cause the processor to perform a method, said method comprising measuring a CARdiac Generation Potential Index of cells which is a function of the quantification of the expression of genes of said cells, wherein at least one gene is selected from the group consisting ofNkx2.5, Tbx5, MEF2C, GATA4, GATA6, Mesp1, and FOG1, and wherein the CARPI is a multivariate equation where the expression of genes is quantified as variables.

Fig. 1

# EP 3 524 980 A1

**EUROPEAN SEARCH REPORT**

Europäisches Patentamt
European Patent Office
Office européen des brevets

Application Number

EP 18 20 8719

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/054819 A2 (GEN HOSPITAL CORP [US]; CHIEN KENNETH R [US] ET AL.) 8 May 2008 (2008-05-08) * [14], [17]-[18], [20], [38], [121], [127], [129], [135], [142], [163], [281], [290], [292], [295], [297], [15], [219],[22], [218] * | 1-10 | INV. G01N33/50 |
| X | US 2003/054973 A1 (ANVERSA PIERO [US]) 20 March 2003 (2003-03-20) * [0081], [0121], [0254], [0271]-[0273], [0308], [0312] * | 1-10 | |
| X | WO 2008/060446 A2 (DAVID GLADSTONE INST [US]; SRIVASTAVA DEEPAK [US]; KWON CHULAN [US]) 22 May 2008 (2008-05-22) * [0079], [0081], [0086], [0038], [0055]; table 1 * | 1-10 | |
| X,P | WO 2009/145761 A1 (MAYO FOUNDATION [US]; TERZIC ANDRE [US]; BEHFAR ATTA [US]) 3 December 2009 (2009-12-03) * p. 1 li. 28-p. 2 li. 22, p. 3 li. 6-10, p. 9 li. 18-23, p. 9 li. 30-p. 10 li. 18, p. 8 li. 29-p. 9 li. 2, p. 9 li. 3-8; example 1 * | 1-10 | TECHNICAL FIELDS SEARCHED (IPC) G01N |

The present search report has been drawn up for all claims

| Place of search | Date of completion of the search | Examiner |
|---|---|---|
| Munich | 15 April 2019 | Landré, Julien |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 524 980 A1**

## ANNEX TO THE EUROPEAN SEARCH REPORT
## ON EUROPEAN PATENT APPLICATION NO.

EP 18 20 8719

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2019

| Patent document cited in search report | | Publication date | Patent family member(s) | | Publication date |
|---|---|---|---|---|---|
| WO 2008054819 | A2 | 08-05-2008 | US | 2010166714 A1 | 01-07-2010 |
| | | | WO | 2008054819 A2 | 08-05-2008 |
| US 2003054973 | A1 | 20-03-2003 | AU | 2003220238 A1 | 22-12-2003 |
| | | | AU | 2009208138 A1 | 03-09-2009 |
| | | | CA | 2488346 A1 | 18-12-2003 |
| | | | EP | 1531865 A2 | 25-05-2005 |
| | | | IL | 215423 A | 30-04-2013 |
| | | | JP | 4685445 B2 | 18-05-2011 |
| | | | JP | 5271953 B2 | 21-08-2013 |
| | | | JP | 2005534651 A | 17-11-2005 |
| | | | JP | 2010195804 A | 09-09-2010 |
| | | | NZ | 561946 A | 30-07-2010 |
| | | | NZ | 586073 A | 28-10-2011 |
| | | | US | 2003054973 A1 | 20-03-2003 |
| | | | US | 2009143296 A1 | 04-06-2009 |
| | | | WO | 03103611 A2 | 18-12-2003 |
| WO 2008060446 | A2 | 22-05-2008 | CN | 101573442 A | 04-11-2009 |
| | | | EP | 2084263 A2 | 05-08-2009 |
| | | | JP | 2010508846 A | 25-03-2010 |
| | | | US | 2010129915 A1 | 27-05-2010 |
| | | | US | 2016194608 A1 | 07-07-2016 |
| | | | WO | 2008060446 A2 | 22-05-2008 |
| WO 2009145761 | A1 | 03-12-2009 | AU | 2009257801 A1 | 17-12-2009 |
| | | | BR | PI0912292 A2 | 20-10-2015 |
| | | | CA | 2724694 A1 | 17-12-2009 |
| | | | CN | 102046193 A | 04-05-2011 |
| | | | CN | 103340902 A | 09-10-2013 |
| | | | EP | 2303310 A2 | 06-04-2011 |
| | | | EP | 2946787 A1 | 25-11-2015 |
| | | | ES | 2556631 T3 | 19-01-2016 |
| | | | HK | 1152225 A1 | 21-02-2014 |
| | | | HK | 1190306 A1 | 05-01-2018 |
| | | | IL | 209555 A | 31-10-2013 |
| | | | IL | 223453 A | 31-12-2013 |
| | | | IL | 228305 A | 31-05-2016 |
| | | | JP | 6093500 B2 | 08-03-2017 |
| | | | JP | 2011521645 A | 28-07-2011 |
| | | | KR | 20110034617 A | 05-04-2011 |
| | | | KR | 20160084501 A | 13-07-2016 |
| | | | NZ | 589415 A | 28-09-2012 |
| | | | NZ | 600920 A | 26-04-2013 |
| | | | RU | 2010153243 A | 10-07-2012 |
| | | | SG | 10201506904T A | 29-10-2015 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 18 20 8719

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2019

| Patent document cited in search report | Publication date | Patent family member(s) | | Publication date |
|---|---|---|---|---|
| | | SG | 10201508604U A | 27-11-2015 |
| | | US | 2011117065 A1 | 19-05-2011 |
| | | US | 2014348803 A1 | 27-11-2014 |
| | | WO | 2009145761 A1 | 03-12-2009 |
| | | WO | 2009151907 A2 | 17-12-2009 |
| | | ZA | 201008046 B | 25-04-2012 |
| | | ZA | 201304418 B | 29-04-2015 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009044751 W
- US 20050164382 A, Chunhui Xu
- US 20020061837 A, Lough
- US 20080019944 A

**Non-patent literature cited in the description**

- **ABDEL-LATIF A. et al.** Adult bone marrow-derived cells for cardiac repair: a systematic review and meta-analysis. *Arch Intern Med.,* 2007, vol. 167, 989-997
- **BEHFAR et al.** Guided stem cell cardiopoietic: Discovery and translation. *J. Mol. and Cell. Cardiology,* 2008, vol. 45, 523-529
- **BEHFAR A. et al.** Derivation of a cardiopoietic population from human mesenchymal stem cells yields cardiac progeny. *Nature Clinical Practice,* 2006, vol. 3, S78-S82
- **BEHFAR et al.** Derivation of a cardiopoietic population from human mesenchymal stem yields progeny. *Nature Clin. Pract., Cardiovasc. Med.,* 2006, vol. 3, S78-S82
- **BEHFAR et al.** Cardiopoietic programming of embryonic stem cells for tumour-free heart repair. *J Exp Med,* 2007, vol. 204, 405-420